Europäisches Patentamt

European Patent Office

Office européen des brevets

(11) Veröffentlichungsnummer : **0 435 826 A1**

(12) # EUROPÄISCHE PATENTANMELDUNG

(21) Anmeldenummer : 90811002.6

(22) Anmeldetag : 18.12.90

(51) Int. Cl.⁵ : **A61K 31/55, A61K 47/48,** A61K 9/18

(30) Priorität : 27.12.89 CH 4653/89

(43) Veröffentlichungstag der Anmeldung :
03.07.91 Patentblatt 91/27

(84) Benannte Vertragsstaaten :
AT BE CH DE DK ES FR GB GR IT LI LU NL SE

(71) Anmelder : CIBA-GEIGY AG
Klybeckstrasse 141
CH-4002 Basel (CH)

(72) Erfinder : Steulet, Anne-Françoise
Vernes 103
CH-2842 Rossemaison (CH)
Erfinder : Schmutz, Markus, Dr.
Baumgartenweg 24
CH-4124 Schönenbuch (CH)
Erfinder : Maître, Laurent, Dr.
Pfeffingerstrasse 99
CH-4053 Basel (CH)
Erfinder : Bernasconi, Raymond, Dr.
Amselstrasse 17A
CH-4104 Oberwil (CH)
Erfinder : Stahl, Peter Heinrich, Dr.
Lerchenstrasse 28
W-7800 Freiburg (DE)

(54) Intravenöse Lösungen für Status Epilepticus.

(57)    Die Erfindung betrifft eine pharmazeutische Zusammensetzung für die intravenöse Applikation von Carbamazepin oder Oxcarbazepin. Die pharmazeutische Zusammensetzung enthält :
a) die Wirkstoffe Carbamazepin oder Oxcarbazepin ;
b) als Löslichkeitsvermittler ein veräthertes, wasserlösliches γ-Cyclodextrinderivat ;
c) die Trägerflüssigkeit und gegebenenfalls weitere wasserlösliche, pharmazeutische Hilfsstoffe.

EP 0 435 826 A1

## INTRAVENÖSE LÖSUNGEN FÜR STATUS EPILEPTICUS

Die Erfindung hat pharmazeutische Zusammensetzungen für die intravenöse Applikation von Carbamazepin, Oxcarbazepin und Metaboliten dieser Verbindungen zum Gegenstand, Trockenpräparate enthaltend diese Verbindungen und einen Löslichkeitsvermittler, Verfahren zur Herstellung der intravenös applizierbaren pharmazeutischen Zusammensetzungen und der Trockenpräparate sowie die Anwendung der pharmazeutischen Zusammensetzungen in einem therapeutischen Verfahren, insbesondere als Antikonvulsiva für Injektionslösungen mit schnellem Wirkungseintritt zur Behandlung von Status Epilepticus oder für Infusionslösungen.

Carbamazepin, 5H-Dibenz[b,f]azepin-5-carboxamid (Tegretol®, Tegretal® :Ciba-Geigy), gilt als Mittel der ersten Wahl bei der Behandlung von Konvulsionen und starken Schmerzzuständen. Die handelsüblichen Darreichungsformen sind für die perorale Verabreichung vorgesehen, z.B. Tabletten mit 200 mg Wirkstoff oder Sirupe mit 2%-igem Wirkstoffgehalt.

Oxcarbazepin, 10,11-Dihydro-10-oxo-5-dibenz[b,f]-acepin-5-carboxamid (Trileptal® :Ciba-Geigy) ist ebenfalls als Antikonvulsivum bekannt. Klinische Befunde für Oxcarbazepin sind in mehreren Publikationen beschrieben, z.B. in The Lancet, July 22, 1989, SS. 196-198 oder in Antiepileptic Drugs, Third Ed., Raven Press N.Y. 1989, Chapter 66, SS. 913-924.

Wegen der geringen Wasserlöslichkeit, siehe Intern. Clinical Psychopharmacology, Jan. 1990, Vol. 5, Supplement, SS. 73-82, siehe S. 74, Table 1, eignen sich beide Präparate nur für enterale Darreichungsformen, z.B. Tabletten, Kapseln oder Sirupe. Solche oralen Darreichungsformen sind prophylaktisch für regelmässig wiederkehrende Verabreichungen verwendbar, um unter Normalbedingungen des Patienten ohne Konvulsionen und bei normalem Bewusstsein eine gleichmässig hohe Wirkstoffkonzentration im Blut zu gewährleisten.

Bei akuten epileptischen Anfällen verbunden mit lebensgefährdenden Krampfartigen Zuständen, z.B. Status Epilepticus, welche Sofortapplikationen mit möglichst raschem Wirkungseintritt zur Beendigung der Krämpfe unbedingt erfordern, sind die oralen Darreichungsformen mit ihrem relativ langsamen Wirkungseintritt ungeeignet. Diese können im Gegensatz zu intravenösen Lösungen während des Krampfanfalls selbst gar nicht appliziert werden. Dasselbe Problem tritt bei sich intensivierenden, lang anhaltenden Schmerzzuständen auf.

Daher benötigt man für den unbedingt erforderlichen Wirkungseintritt innerhalb kurzer Zeit Injektionslösungen, welche intravenös appliziert werden.

Infusionslösungen werden in der Klinik benötigt, um das Auftreten von epileptischen Anfällen mit Konvulsionen bei Patienten ohne Bewusstsein (prae- oder postoperativ) oder vermindertem Bewusstsein (Ruhephasen während der Behandlung) zu verhindern.

Vor allem sind die durch plötzliche Anfälle verursachten Zwischenfälle bei chirurgischen Eingriffen für Epilepsie-gefährdete Patienten besonders gefährlich.

Intravenöse Darreichungsformen (Injektions- und Infusionslösungen) setzen jedoch die Wasserlöslichkeit von therapeutisch wirksamen Mengen des zu applizierenden Wirkstoffs voraus. Wegen ihrer geringen Wasserlöslichkeit kann die therapeutische Mindestdosis der beiden Wirkstoffe Carbamazepin und Oxcarbazepin nicht in gelöstem Zustand, sondern nur als Suspension mit Feststoffpartikeln vorliegen, deren intravenöse Applikation unzulässig ist.

Zur Verbesserung der Löslichkeit sind verschiedene Löslichkeitsvermittler bekannt, z.B. hydrophile Cosolventien wie Ethanol, Glycerin, Propylenglycol, flüssige Polyethylenglycole oder lipophile Löslichkeitsvermittler wie Lecithin, Fettsäurepolyglycolester oder Fettsäureglycerinpolyglycolester. Die Verwendung solcher Löslichkeitsvermittler ist wegen mangelnder Verträglichkeit, z.B. Gefahr von Embolien, und fehlender Stabilität, z.B. Entmischungseffekten bei Lagerung der parenteralen Lösung, generell problematisch.

Der vorliegenden Erfindung liegt die Aufgabe zugrunde, für die Antiepileptika mit geringer Wasserlöslichkeit, Carbamazepin und Oxcarbazepin, intravenöse Darreichungsformen mit schnellem Wirkungseintritt, guter Verträglichkeit und hoher Stabilität bereitzustellen, welche für die genannten epileptischen Anfälle bei gefährdeten Patienten, insbesondere Status Epilepticus, entweder unmittelbar nach ihrem Auftreten der vorsorglich bei chirurgischen Eingriffen verwendbar sind.

Es wurde überraschenderweise gefunden, dass bei Verwendung von verätherten γ-Cyclodextrinderivaten, insbesondere Hydroxypropyl-γ-cyclodextrin, die Wasserlöslichkeit von Carbamazepin und Oxcarbazepin sich steigern lässt und dabei diese Wirkstoffe in einer für intravenöse Applikationen geeigneten therapeutischen Menge in Wasser lösbar oder zumindest kolloidal dispergierbar sind.

Dieser überraschende Befund ist zur Lösung der Aufgabenstellung verwendbar, welche der vorliegenden Erfindung zugrunde liegt. Die Erfindung betrifft folglich eine pharmazeutische Zusammensetzung für die intravenöse Applikation der in Wasser schwerlöslichen, antiepileptisch wirksamen Verbindungen Carbamazepin und Oxcarbazepin enthaltend :

a) die in Wasser schwerlöslichen antiepileptisch wirksamen Verbindungen Carbamazepin oder Oxcarbazepin sowie Metabolite davon ;

b) als Löslichkeitsvermittler ein veräthertes wasserlösliches γ-Cyclodextrinderivat ;

c) die für Injektionszwecke zubereitete Trägerflüssigkeit und gegebenenfalls weitere für die Herstellung von intravenösen Darreichungsformen geeignete Hilfsstoffe.

Diese pharmazeutische Zusammensetzung zeichnet sich durch besondere Stabilität aus. So wird in einem Zeitraum bis zu mehreren Monaten keine Sedimentation, z.B. von Wirkstoffkristallen und/oder Hilfsstoffpartikeln, beobachtet. Die Zusammensetzung eignet sich ausserdem für die Herstellung einer intravenösen Darreichungsform mit einer therapeutisch wirksamen Dosis des betreffenden schwerlöslichen Antiepileptikums sowohl in einem relativ geringen Volumen, z.B. 2-10 ml, Trägerflüssigkeit als auch in grossen Volumina bis zu ca. 1,5 l für Infusionslösungen.

Die weiter vom und im folgenden verwendeten allgemeinen Definitionen und Begriffe haben im Rahmen der Beschreibung der Erfindung vorzugsweise die folgenden Bedeutungen :

Der Begriff pharmazeutische Zusammensetzung umfasst eine applikationsfähige Mischung bestehend aus dem für die Verabreichung vorgesehenen Wirkstoff in einer höheren Dosis als der therapeutischen Mindestdosis, der Trägerflüssigkeit und den für die betreffende Darreichungsform z.B. Injektions- oder Infusionslösung, unter Berücksichtigung der Applikationsart - hier i.v. - geeigneten Hilfsstoffen.

Der Begriff intravenöse Applikation definiert die therapeutische Massnahme der Zufuhr einer Injektions- oder Infusionslösung des betreffenden Wirkstoffes in eine Vene des Patienten und damit direkt ins Blut und zwar aufgrund einer akuten Indikationsstellung, im vorliegenden Fall Konvulsionen als Folge von epileptischer Hirnaktivität, Anfällen oder starken Schmerzzuständen oder des Risikos von Konvulsionen während und nach chirurgischen Eingriffen.

Ein Metabolit von Carbamazepin und Oxcarbazepin ist z.B. 10-Hydroxy-10,11-dihydro-5H-dibenz[b,f]azepin-5-carboxamid. Diese Verbindung ist als Metabolit von Oxcarbazepin nachgewiesen und existiert sowohl als racemische Mischung seiner R- und S-Enantiomere als auch in Form seiner optisch reinen, individuellen R- und S-Enantiomere.

Der Begriff Löslichkeitsvermittler definiert einen Hilfsstoff oder ein Hilfsstoffgemisch, welches die Dispersionsfähigkeit eines in Wasser schwerlöslichen Wirkstoffs oder Wirkstoffgemischs soweit erhöht, dass therapeutisch wirksame Dosen des Wirkstoffs oder Wirkstoffgemisches gelöst oder zumindest kolloidal - im vorliegenden Fall i.v. - applizierbar sind. Der Begriff Dispersionsfähigkeit definiert ein Mass für die Bildung von echten molekularen Lösungen der Wirkstoffe und der Hilfsstoffe in Wasser sowie kolloidalen Lösungen, z.B. Lösungen aus Assoziationskolloiden oder Molekülkolloiden, die klar bzw. opaleszierend sind und gegebenenfalls nach Filtrieren, insbesondere mit sterilen Filtern und ca. 5-10 μm Porendurchmesser, keinerlei Feststoffpartikel aufweisen, z.B. mizellare Lösungen oder Sphärokolloide, die nur in der Ultrazentrifuge abtrennbar sind. Die Dispersionsfähigkeit kann z.B. in mg oder mMol Wirkstoff pro Liter Wasser angegeben werden.

Die Aethergruppen des verätherten γ-Cyclodextrinderivats sind vorzugsweise wie folgt definiert :

$C_1$-$C_6$-Alkyl ist vorzugsweise Methyl oder Aethyl.

Carboxy-$C_1$-$C_6$-alkyl ist vorzugsweise Carboxymethyl, wobei die Carboxygruppe in Salzform, z.B. als Natriumcarboxylatgruppe, vorliegen kann.

$C_1$-$C_6$-Alkoxycarbonyl-$C_1$-$C_6$-alkyl ist vorzugsweise Methoxy- oder Aethoxycarbonylmethyl.

Hydroxy-$C_2$-$C_6$-alkyl, worin die Hydroxygruppe sich in einer höheren als der 1-Stellung des $C_2$-$C_4$-Alkylrests befindet, ist insbesondere 2-Hydroxyäthyl oder 2- oder 3-Hydroxypropyl.

Durch $C_1$-$C_6$-Alkyl, Carboxy-$C_1$-$C_6$-alkyl, $C_1$-$C_6$-Alkoxycarbonyl-$C_1$-$C_6$-alkyl und/oder Hydroxy-$C_2$-$C_6$-alkyl, wobei die Hydroxygruppe sich in einer höheren als der 1-Stellung des $C_2$-$C_4$-Alkylrests befindet, veräthertes γ-Cyclodextrin ist z.B. ein γ-Cyclodextrinderivat mit 8 Anhydroglucoseeinheiten, welches an einer primären Hydroxygruppe der Anhydroglucoseeinheit durch $C_1$-$C_6$-Alkyl, z.B. Methyl oder Aethyl, ferner Carboxyalkyl, z.B. Carboxymethyl, oder $C_1$-$C_6$-Alkoxycarbonyl-$C_1$-$C_6$-alkyl, z.B. Aethoxycarbonylmethyl, und an ein bis zwei sekundären Hydroxygruppen durch Hydroxy-$C_2$-$C_4$-alkyl, z.B. 2-Hydroxyäthyl oder 2- oder 3-Hydroxypropyl, veräthert ist, z.B. 2- oder 3-Hydroxypropyl-methyl-γ-cyclodextrin oder 2-Hydroxyäthyl-methyl-γ-cyclodextrin.

Bevorzugt sind die auschliesslich durch Hydroxy-$C_2$-$C_4$-alkyl substituierten γ-Cyclodextrinderivate, z.B. 2-Hydroxypropyl- oder 3-Hydroxypropyl-γ-cyclodextrin oder 2-Hydroxyäthyl-γ-cyclodextrin.

γ-Cyclodextrin selbst ist eine bekannte cyclische Verbindung mit 8 Anhydroglucoseeinheiten (Cyclooctaamylose). Jede Anhydroglucoseeinheit ist in 2-, 3- und 6-Stellung durch Hydroxygruppen substituiert, welche sich nur partiell und daher nicht vollständig veräthern lassen. Der Grad der Substitution oder Verätherung durch $C_1$-$C_4$-Alkylgruppen, $C_1$-$C_6$-Alkoxycarbonyl-$C_1$-$C_6$-alkyl- oder Carboxy-$C_1$-$C_6$-alkylgruppen wird durch die Kennzahl DS = Durchschnittlicher Substitutionsgrad angegeben. Diese Kennzahl ist ein gemittelter Wert der verätherten Hydroxygruppen pro Anhydroglucoseeinheit. Der DS-Wert von geeigneten Derivaten beträgt ca.

0,05 bis 2,0, vorzugsweise ca. 0,2 bis 1,5. Der Bereich von 0,5 bis 1,2 ist besonders bevorzugt.

Der Grad der Substitution oder Verätherung durch die genannten Hydroxy-$C_2$-$C_4$-alkyl-gruppen wird durch die Kennzahl MS = Molarer Substitutionsgrad angegeben. Diese Kennzahl gibt die Anzahl der Mole des zweiten Alkylierungsmittels an, z.B. Propylenoxid oder Aethylenoxid, welche bei der Herstellung des verätherten γ-Cyclodextrinderivats verwendet worden sind. Wegen der Substitutionsneigung der gebildeten Hydroxygruppe in der Seitenkette bei möglicher weiterer Polymerisation und dem dadurch bedingten erhöhten Verbrauch an Alkylierungsmittel kann der MS-Wert höher als der DS-Wert sein.

Der MS-Wert von geeigneten Derivaten beträgt ca. 0,05 bis 10, vorzugsweise ca. 0,2 bis 2. Besonders bevorzugt ist der MS-Wert von ca. 0,25 bis 1.

Die Herstellung der genannten γ-Cyclodextrinderivate ist bekannt oder kann in an sich bekannter Weise erfolgen. So können γ-Cyclodextrinderivate, welche durch $C_1$-$C_4$-Alkyl und Hydroxy-$C_2$-$C_4$-alkyl substituiert sind, durch Umsetzung von γ-Cyclodextrin mit mindestens einem Aequivalent Alkoholat, z.B. Natriummethanolat, in einem aprotischen Lösungsmittel, z.B. Dimethylformamid, Acetonitril oder Dimethoxyäthan, und anschliessende Umsetzung mit ein bis zwei Aequivalenten des zweiten Alkylierungsmittels, z.B. Aethylen- oder Propylenoxid, hergestellt werden. Die Herstellung der $C_1$-$C_6$-Alkoxycarbonyl- oder Carboxy-$C_1$-$C_6$-alkyl-Derivate erfolgt analog den für Celluloseäther bekannten Verätherungsreaktionen. Bei Herstellung von Hydroxyäthyl- oder Hydroxypropyl-β-cyclodextrin geht man einstufig vor und setzt nur mit Aethylen- oder Propylenoxid um, z.B. nach dem in der U.S. Patentschrift 3,459,731 beschriebenen Verfahren oder analog der Methode von J. Szejtli et al., Stärke 32, 165 (1980) oder gem. der Methode nach A.P. Croft und R.A. Bartsch, Tetrahedron 39, 1417 (1983).

Besonders bevorzugt sind Hydroxypropyl-γ-cyclodextrin-Derivate mit einem MS-Wert von ca. 0,9, einem durchschnittlichen Molekulargewicht von ca. 1668-1761, einem Wassergehalt von bis zu 7 % (Gewichtsverlust nach Trocknen), einem Gehalt von unsubstituiertem γ-Cyclodextrin von bis zu 2 %, Asche bis zu 0,1 %, Propylenglycol-Rückstand bis zu 2,0 %, org. Lösungsmittelrückstand bis zu 0,1 %, einer minimalen Wasserlöslichkeit von ca. 50 g/100 ml (25°) und einer Löslichkeit in den organischen Lösungsmitteln Methanol, Aethanol, Dimethylformamid, Dimethylsulfoxid, Pyridin.

Bevorzugt sind Hydroxypropyl-γ-cyclodextrinderivate mit der Hydroxygruppe in 2- oder 3-Stellung des Hydroxypropylrests und mit einem MS-Wert von ca. 0,25-1,0 und einer mittleren Molmasse von 1300-1600.

Diese Derivate sind z.B. bei der Firma Wacker bzw. Consortium für Elektrochemische Industrie, D-8000 München, kommerziell erhältlich.

Die für Injektionszwecke zubereitete Trägerflüssigkeit ist eine sterile, pyrogenfreie wässrige Lösung, welche den Wirkstoff in therapeutischer wirksamer Dosis, den weiter vorn beschriebenen Lösungsvermittler und gegebenenfalls weitere für die Herstellung von Injektionslösungen geeignete Hilfsstoffe enthält.

Bezüglich der Forderungen "steril" und "pyrogenfrei" wird auf die in nationalen Pharmakopien bzw. der Europäischen Pharmakopie publizierten Vorschriften verwiesen, welche bei Wasser für Injektionszwecke maximale Keimzahlen von weniger als 10 000 Keimen pro Liter vorsehen. Die Herstellung der entsprechenden Bedingungen entspricht gängiger pharmazeutischer Praxis, z.B. Elektrodialyse oder Kombination von Gegenosmose, Ionenaustausch, Aktivkohlefiltration und Entkeimungsfiltration durch Membranen.

Für die Herstellung von Injektionslösungen geeignete weitere Hilfsstoffe sind z.B. wasserlösliche ionische Zusätze wie Kochsalz oder Glycin. Insbesondere sind die genannten wasserlöslichen Zusätze in den vorgeschriebenen Mengen enthalten, welche für die Herstellung von isotonischen Bedingungen der intravenösen Lösungen erforderlich sind. Zur Herstellung von isotonischen Bedingugnen eignen sich ausserdem wasserlösliche, physiologisch inerte Verbindungen wie Mannit, Glucose, Sorbit etc.

Für die Herstellung von Injektionslösungen geeignete weitere Hilfsstoffe sind ausserdem die bekannten für flüssige pharmazeutische Formulierungen verwendbaren Netzmittel oder Tenside, insbesondere nichtionische Tenside vom Typ Fettsäure-Polyhydroxyalkoholester wie Sorbitanmonolaurat, -oelat, -stearat oder -palmitat, Sorbitantristearat oder -trioleat, Polyoxyäthylen-Addukte von Fettsäure-Polyhydroxyalkoholestern wie Polyoxyäthylen-sorbitanmonolaurat, -oleat, -stearat, -palmitat -tristearat oder -trioleat oder Polyäthylenglycol-Fettsäureester wie Polyoxyäthylstearat, Polyäthylenclycol-400-stearat oder Polyäthylenglycol-2000-stearat, insbesondere Aethylenoxid-Propylenoxid Blockpolymere vom Typ Pluronic® (Wyandotte Chem. Corp.) oder Synperonic® (ICI).

Die genannten Tenside können in Gewichtsmengenverhältnissen von Wirkstoff zu Tensid von ca. 1 :0,001 bis 1 :0,1, vorzugsweise von ca. 1 :0,03 bis 1 :0,1, in der intravenösen Formulierung vorhanden sein.

Die vorliegende Erfindung betrifft vorzugsweise eine pharmazeutische Zusammensetzung enthaltend

a) Carbamazepin oder Oxcarbazepin ;

b) als Löslichkeitsvermittler durch $C_1$-$C_6$-Alkyl, Carboxy-$C_1$-$C_6$-alkyl, $C_1$-$C_6$-Alkoxycarbonyl-$C_1$-$C_6$-alkyl und/oder Hydroxy-$C_2$-$C_6$-alkyl, wobei die Hydroxygruppe in einer höheren als der 1-Stellung des $C_2$-$C_4$-Alkylrests sich befindet, veräthertes γ-Cyclodextrin ;

4

c) die Trägerflüssigkeit und gegebenenfalls weitere wasserlösliche, pharmazeutische Hilfsstoffe.

Die vorliegende Erfindung betrifft vorzugsweise eine pharmazeutische Zusammensetzung enthaltend

a) Carbamazepin oder Oxcarbazepin ;

b) als Löslichkeitsvermittler durch Hydroxy-$C_2$-$C_4$-alkyl, wobei die Hydroxygruppe sich in einer höheren als der 1-Stellung des $C_2$-$C_4$-Alkylrests befindet, veräthertes γ-Cyclodextrin ;

c) die Trägerflüssigkeit und gegebenenfalls weitere wasserlösliche, pharmazeutische Hilfsstoffe.

Die Erfindung betrifft vor allem eine pharmazeutische Zusammensetzung enthaltend :

a) Carbamazepin oder Oxcarbazepin ;

b) als Löslichkeitsvermittler Hydroxypropyl-γ-cyclodextrin, worin die Hydroxygruppe des Hydroxypropylrests sich in 2- oder in 3-Stellung dieses Rests befindet ;

c) die wässrige Trägerflüssigkeit und gegebenenfalls weitere wasserlösliche pharmazeutische Hilfsstoffe.

Die Erfindung betrifft ausserdem das Trockenpräparat bestehend aus der Einschlussverbindung von Carbamazepin oder Oxcarbazepin, dem verätherten, wasserlöslichen γ-Cyclodextrinderivat und gegebenenfalls für die Herstellung von Trockenpräparaten geeigneten wasserlöslichen Hilfsstoffen.

Bevorzugt ist das Trockenpräparat bestehend aus der Einschlussverbindung von Carbamazepin oder Oxcarbazepin in Hydroxypropyl-γ-cyclodextrin, worin die Hydroxygruppe des Hydroxypropylrests sich in 2- oder 3-Stellung dieses Rests befindet.

Die Erfindung betrifft ebenfalls ein Verfahren zur Herstellung einer pharmazeutischen Zusammensetzung für die intravenöse Applikation der in Wasser schwerlöslichen, antiepileptisch wirksamen Verbindungen Carbamazepin und Oxcarbazepin, welches dadurch gekennzeichnet ist, dass man

α) als Löslichkeitsvermittler ein veräthertes, wasserlösliches γ-Cyclodextrinderivat in der Trägerflüssigkeit löst, Carbamazepin, Oxcarbazepin oder einen Metaboliten davon und gegebenenfalls weitere wasserlösliche, pharmazeutische Hilfsstoffe zur Trägerflüssigkeit zusetzt und gegebenenfalls die Lösung homogenisiert ; oder

β) ein Trockenpräparat enthaltend Carbamazepin, Oxcarbazepin oder einen Metaboliten davon und das verätherte, wasserlösliche γ-Cyclodextrinderivat herstellt und gegebenenfalls für die Herstellung von Trockenpräparaten geeignete wasserlösliche Hilfsstoffe hinzufügt und das Trockenpräparat in der Trägerflüssigkeit auflöst.

## Verfahren α

Nach dieser Variante wird pyrogenfreies und gegebenenfalls sterilfiltriertes Wasser vorgelegt und gegebenenfalls unter Erwärmen, z.B. in einem Temperaturbereich von etwa 20° bis etwa 100°C, vorzugsweise 40-60°C, der Löslichkeitsvermittler, insbesondere das Hydroxypropyl-γ-cyclodextrin, sowie die weiteren Hilfsstoffe zugegeben. Zu dieser Grundlage wird anschliessend der Wirkstoff, insbesondere gemahlenes, wasserfreies Carbamazepin, gegeben. Der Lösungsvorgang kann durch abschliessendes Homogenisieren, z.B. durch Behandeln mit Ultraschall oder hochtouriges Rühren, z.B. mit einem Schnellrührer der Fa. Vortex, beschleunigt bzw. vervollständigt werden. Es wird eine klare Lösung erhalten, die keinerlei Aggregate enthält und homogen ist. So wird keine Lichtstreuung im Sinne eines Tyndalleffekts beobachtet.

Die Lösung lässt sich je nach Wirkstoffgehalt in Ampullen oder Injektionsfläschchen mit Volumina von ca. 1 ml bis ca. 50 ml abfüllen, die dann gegebenenfalls im Autoklaven hitzesterilisiert (120°C/20 min) werden, oder sie wird sterilfiltriert und unter aseptischen Bedingungen, beispielsweise in Behälter für Infusionslösungen, abgefüllt.

## Verfahren β

Das erfindungsgemässe Trockenpräparat wird hergestellt, indem man z.B. die für die intravenöse Applikation vorgesehene Menge des Wirkstoffs in mikronisierter Form in einem Suspensionsmedium, z.B. pyrogenfreiem Wasser, welches den Löslichkeitsvermittler und gegebenenfalls die pharmazeutisch annehmbaren Zusatzstoffe (Hilfsstoffe) enthält, suspendiert und das Lösungsmittel entfernt.

Das Trockenpräparat kann wasserlösliche Zusätze oder Hilfsstoffe wie Kochsalz, Mannit oder Glucose enthalten, welche z.B. zur Herstellung von isotonischen Bedingungen erforderlich sind. Nach dem Auflösen der Zusatzstoffe in Wasser zur Injektion wird der Wirkstoff zugegeben und gelöst. Nach Sterilfiltration kann die Herstellung des Trockenpräparats durch Eindampfen oder mittels bekannter Gefriertrocknungsmethoden erfolgen, z.B. indem man eine bestimmte Menge der hergestellten Suspension üblicherweise in geeignete Behälter, wie Ampullen, z.B. Glasstechampullen (Vials), abfüllt und die abgefüllten Stechampullen bei ca. -40° bis -50°C einfriert und anschliessend bei einem Druck von ca. 0,2-0,6 mbar durch langsames Erwärmen in einem Zeitraum von ca. 24-48 h bis zu einer Endtemperatur von ca. 25°-35°C lyophilisiert.

5

Ueberraschenderweise gelingt es mit den genannten Verfahren, Trockenpräparate, insbesondere Lyophilisate, und daraus rekonstituierbare Lösungen herzustellen, welche stabil und zur Injektion geeignet sind.

Die Verwendung eines verätherten, wasserlöslichen γ-Cyclodextrinderivats zur Herstellung einer wässrigen, intravenös applizierbaren Lösung gemäss einer der beiden Verfahrensvarianten ist ebenfalls Gegenstand der vorliegenden Erfindung.

Die Verwendung des gemäss den genannten Verfahren erhältlichen Trockenpräparates zur Herstellung von Injektionslösungen ist ebenfalls Gegenstand der vorliegenden Erfindung. Diese Injektionslösungen können intravenös appliziert werden.

Das erfindungsgemäss erhältliche Trockenpräparat wird vorzugsweise unmittelbar vor der Applikation als klare Lösung in der vorgesehenen Flüssigkeitsmenge, insbesondere sterilem Wasser zur Injektion, rekonstituiert.

Durch Aufschütteln bildet sich wieder die homogene Lösung des Wirkstoffs. Statt eines Trockenpräparats enthaltend den Wirkstoff und sämtliche übrigen Zusatzstoffe wie Kochsalz oder Mannit lässt sich auch ein Trockenpräparat enthaltend nur den Wirkstoff und Löslichkeitsvermittler b) - ohne Zusatzstoffe c) - lösen. Diese Variante eignet sich zur Herstellung von Infusionslösungen.

Die pharmazeutischen Zusammensetzungen zeichnen sich durch gute Lagerstabilität aus. Es wird keine Abscheidung von Niederschlägen oder Kristallen aus der Lösung beobachtet.

In einer besonders bevorzugten Ausführungsform werden Injektionslösungen, die die üblichen Dosen von 10-250 mg Wirkstoff enthalten, mit einem Gesamtvolumen von 1,0-50 ml, insbesondere 2,0-10,0 ml, hergestellt.

Diese Lösungen können als "ready to use" Präparate verwendet werden.

Die erfindungsgemässe intravenös applizierbare Darreichungsform besitzt wertvolle pharmakologische Eigenschaften und kann als Antineuralgikum und insbesondere als Antikonvulsivum zur Behandlung von starken Krampfzuständen und Konvulsionen, insbesondere Konvulsionen vom Typ Status Epilepticus, sowie zum prophylaktischen Schutz prädisponierter Personen gegen epileptisch auftretende Konvulsionen in Situationen, wobei die sonst übliche perorale Verabreichung nicht erfolgen kann, etwa bei Bewusstlosigkeit, Lähmungen, nach Unfällen oder bei Operationen, verwendet werden. Die Verwendung dieser Darreichungsform zur Behandlung solcher Krankheiten, insbesondere der Epilepsie, ist ebenfalls Gegenstand der vorliegenden Erfindung.

Die Erfindung betrifft insbesondere auch die in den Beispielen beschriebenen Zubereitungen bzw. Herstellungsverfahren.

Die nachfolgenden Beispiele dienen lediglich der Erläuterung der vorstehend beschriebenen Erfindung ; sie sollen diese jedoch in ihrem Umfang in keiner Weise einschränken.

Beispiel 1 : Zur Herstellung einer Carbamazepin enthaltenden Injektionslösung werden 100,0 g Hydroxypropyl-γ-cyclodextrin (MS = 0,6) in 100 ml pyrogenfreiem, bidestilliertem Wasser gelöst. In 100 ml dieser Lösung werden 1500 mg Carbamazepin durch Rühren bei Raumtemperatur (= 20°C) gelöst. Die Lösung wird filtriert, in Ampullen zu je 10,0 ml abgefüllt und in einem Autoklaven 20 Minuten bei 120°C sterilisiert.

Die Lösung hat folgende Eigenschaften :

Gehalt :     150 mg Carbamazepin in 10,0 ml

Aussehen :   klare, farblose Lösung

pH-Wert :    6,5-7,0

Beispiel 2 : In ähnlicher Weise wie in Beispiel 1 werden zur Herstellung einer Oxcarbazepin enthaltenden Injektionslösung 100,0 g Hydroxypropyl-γ-cyclodextrin (MS = 0,6) in 100 ml pyrogenfreiem, bidestilliertem Wasser gelöst. In 100 ml dieser Lösung werden 400,0 mg fein gemahlenes Oxcarbazepin durch Rühren bei 22°C gelöst. Die Lösung wird filtriert, in Ampullen zu je 10 ml abgefüllt und in einem Autoklaven 20 Minuten bei 120°C sterilisiert.

Die Lösung hat folgende Eigenschaften :

Gehalt :     40 mg Oxcarbazepin in 10,0 ml

Aussehen :   klare, farblose Lösung

pH-Wert :    6,5-7,0

Beispiel 3 : Zur Vorbereitung von Verdünnungsreihen für in-vivo Versuche wird die 50%-ige Lösung von Hydroxypropyl-γ-cyclodextrin mit Wasser verdünnt. Die Löslichkeit bei 22°C von Oxcarbazepin in einer 40%-igen wässrigen Lösung von Hydroxypropyl-γ-cyclodextrin beträgt :

Gehalt :     37 mg Oxcarbazepin in 10,0 ml

Aussehen :   klare, farblose Lösung

Diese Lösung von Oxcarbazepin in 40%-igem wässrigen Hydroxypropyl-γ-cyclodextrin wird für die Bestimmung der antikonvulsiven Wirkung verwendet.

Beispiel 4 : In ähnlicher Weise wie in Beispiel 3 werden 1000 ml einer Lösung aus 200,0 g Hydroxypropyl-γ-cyclodextrin (MS = 0,6) und 1,59 g Oxcarbazepin in pyrogenfreiem, bidestilliertem Wasser hergestellt.

Die Lösung hat folgende Eigenschaften :
Gehalt : 15,9 mg Oxcarbazepin in 10,0 ml
Aussehen : klare, farblose Lösung
pH-Wert : 6,5-7,0

Beispiel 5 : Die akute Schutzwirkung von Carbamazepin und Oxcarbazepin nach intravenöser Verabreichung im Vergleich zur oralen wurde mit Hilfe des maximalen Elektroschocks ermittelt. Die folgenden Methoden wurden verwendet :

-Elektroschock Test an Ratten :

Tonische Konvulsionen der Hinterextremitäten wurden durch Verabreichung eines Wechselstroms von 50 Hz und 36 mA während 0,63 Sekunden mit Hilfe von Cornealelektroden ausgelöst. Es wurden 10 Ratten pro Dosis und Zeiteinheit verwendet ; eine Gruppe diente als Kontrolle. Die Anzahl der Ratten, die keine tonische Konvulsionen der Hinterextremitäten aufwiesen, wurde für jede Gruppe bestimmt. Die $ED_{50}$ wurde mit Hilfe der Regressionsanalyse nach der Methode von Spearman und Kärber berechnet.

-Elektroschock Test an Mäusen :

Tonische Konvulsionen der Hinterextremitäten wurden durch Verabreichung eines Wechselstromes von 50 Hz und 18 mA während 0,2 Sekunden mittels Cornealelektroden ausgelöst. Für jede Dosis und Zeiteinheit wurden 10 Mäuse verwendet ; eine Gruppe diente als Kontrolle. Die Anzahl der Mäuse, die keine tonische Konvulsionen der Hinterextremitäten zeigte, wurde für jede Gruppe bestimmt. Die $ED_{50}$ wurde mit Hilfe der Regressionsanalyse nach der Methode von Spearman und Kärber bestimmt. Die Injektionsvolumina für i.v. Verabreichung sind 5 ml/kg bei Mäusen und 1 ml/kg bei Ratten.

Beispiel 6 : Die pharmakologischen Eigenschaften der verschiedenen Lösungen sind in den Tabellen 1 bis 5 dargestellt. Die antikonvulsive Wirkung von Carbamazepin und Oxcarbazepin tritt nach der intravenösen schneller als nach der oralen Applikation auf (Tabellen 4 und 5). Ueberraschenderweise sind die $ED_{50}$ nach intravenöser Verabreichung wesentlich kleiner als nach oraler Verabreichung (Tabellen 4 und 5).

Die $ED_{50}$ von Carbamazepin und Oxcarbazepin für die Schutzwirkung gegenüber dem maximalen Elektroschock in der Ratte sind in der Tabelle 1 angegeben.

## Tabelle 1:

| Substanzen | $ED_{50}$ in mg/kg |
|---|---|
| Carbamazepin | 2,5 |
| Oxcarbazepin | 2,5 |

Die Antikonvulsiva wurden intravenös 15 Minuten vor den Elektroschocks injiziert. Die $ED_{50}$-Werte wurden durch Regressionsanalyse aus drei verschiedenen Dosen (10 Ratten pro Dosis) berechnet.

Der Zeitverlauf der Schutzwirkung von Carbamazepin nach intravenöser Verabreichung an Ratten ist in der Tabelle 2 dargestellt.

## Tabelle 2:

| Zeit in Minuten | Schutz vor dem maximalen Elektroschock $ED_{50}$ in mg/kg |
|---|---|
| 5 | 2,5 |
| 15 | 2,5 |
| 60 | 3,1 |

Die $ED_{50}$-Werte werden durch Regressionsanalyse aus drei verschiedenen Dosen (10 Ratten pro Dosis) berechnet.

Tabelle 3 stellt den Zeitverlauf der Schutzwirkung von Oxcarbazepin nach intravenöser Verabreichung an

Mäusen ($ED_{50}$) dar.

Tabelle 3:

| Zeit in Minuten | Schutz vor dem maximalen Elektroschock $ED_{50}$ in mg/kg |
|---|---|
| 2,5 | 4,35 |
| 5 | 4,35 |
| 15 | 5,0 |
| 60 | 10,7 |
| 120 | 20 % bei 20 mg/kg |

Die $ED_{50}$-Werte werden durch Regressionsanalyse aus drei verschiedenen Dosen (10 Mäuse pro Dosis) berechnet.

Tabelle 4 stellt den Vergleich der Schutzwirkung von Carbamazepin nach intravenöser und oraler Verabreichung dar.

Tabelle 4:

| Zeit in Minuten | Schutz vor dem maximalen Elektroschock $ED_{50}$ in mg/kg | | | |
|---|---|---|---|---|
| | intravenös | | oral | |
| | Mäuse | Ratten | Mäuse | Ratten |
| 2,5 | 2,9 | | 10 % bei 60 mg/kg | 60,0 |
| 5,0 | 2,6 | 2,5 | 22,7 | 60,0 |
| 15,0 | 4,7 | 2,5 | 22,3 | 15,4 |
| 30 | 6,8 | 2,0 | 10,7 | 9,6 |
| 60 | 6,5 | 3,1 | 13,0 | 10,0 |
| 120 | 17,3 | 4,3 | 27,0 | 12,5 |

Die $ED_{50}$-Werte werden durch Regressionsanalyse aus drei verschiedenen Dosen (10 Tiere pro Dosis) berechnet. Bei der oralen Verabreichung von Carbamazepin wurde eine Suspension in Methocel® verwendet.

Tabelle 5 stellt den Vergleich der Schutzwirkung von Oxcarbazepin nach intravenöser und oraler Verabreichung dar.

Tabelle 5:

| Zeit in Minuten | Schutz vor dem maximalen Elektroschock $ED_{50}$ in mg/kg | | | |
| --- | --- | --- | --- | --- |
| | intravenös | | oral | |
| | Mäuse | Ratten | Mäuse | Ratten |
| 2,5 | 2,35 | — | — | — |
| 5,0 | 4,35 | 3,44 | 40,44 | 20 % bei 30 mg/kg |
| 15,0 | 5,00 | 3,44 | 14,48 | 12,18 |
| 30,0 | 7,07 | 3,44 | 15,74 | 12,18 |
| 60,0 | 10,72 | 4,85 | 17,77 | 15,85 |
| 120 | 20 % bei 20 mg/kg | 5,70 | 36,3 | 16,08 |

Die $ED_{50}$-Werte werden durch Regressionsanalyse aus drei verschiedenen Dosen (10 Tiere pro Dosis) berechnet. Bei der oralen Verabreichung von Oxcarbazepin wurde eine Suspension in Methocel® verwendet.

**Patentansprüche**

1.  Pharmazeutische Zusammensetzung für die intravenöse Applikation der in Wasser schwerlöslichen, antiepileptisch wirksamen Verbindungen Carbamazepin und Oxcarbazepin enthaltend :
    a) die in Wasser schwerlöslichen antiepileptisch wirksamen Verbindungen Carbamazepin und Oxcarbazepin sowie Metabolite davon ;
    b) als Löslichkeitsvermittler ein veräthertes wasserlösliches $\gamma$-Cyclodextrinderivat ;
    c) die für Injektionszwecke zubereitete Trägerflüssigkeit und gegebenenfalls weitere für die Herstellung von intravenösen Darreichungsformen geeignete Hilfsstoffe.

2.  Pharmazeutische Zusammensetzung gemäss Anspruch 1 enthaltend :
    a) Carbamazepin oder Oxcarbazepin ;
    b) als Löslichkeitsvermittler durch $C_1$-$C_6$-Alkyl, Carboxy-$C_1$-$C_6$-alkyl, $C_1$-$C_6$-Alkoxycarbonyl-$C_1$-$C_6$-alkyl und/oder Hydroxy-$C_2$-$C_6$-alkyl, wobei die Hydroxygruppe in einer höheren als der 1-Stellung des $C_2$-$C_4$-Alkylrests sich befindet, veräthertes $\gamma$-Cyclodextrin ;
    c) die Trägerflüssigkeit und gegebenenfalls weitere wasserlösliche, pharmazeutische Hilfsstoffe.

3.  Pharmazeutische Zusammensetzung gemäss Anspruch 1 enthaltend :
    a) Carbamazepin oder Oxcarbazepin ;
    b) als Löslichkeitsvermittler durch Hydroxy-$C_2$-$C_4$-alkyl, wobei die Hydroxygruppe sich in einer höheren als der 1-Stellung des $C_2$-$C_4$-Alkylrests befindet, veräthertes $\gamma$-Cyclodextrin ;
    c) die Trägerflüssigkeit und gegebenenfalls weitere wasserlösliche, pharmazeutische Hilfsstoffe.

4.  Pharmazeutische Zusammensetzung gemäss Anspruch 1 enthaltend :
    a) Carbamazepin oder Oxcarbazepin ;
    b) als Löslichkeitsvermittler Hydroxypropyl-$\gamma$-cyclodextrin, worin die Hydroxygruppe des Hydroxypropylrests sich in 2- oder in 3-Stellung dieses Rests befindet ;
    c) die Trägerflüssigkeit und gegebenenfalls weitere wasserlösliche pharmazeutische Hilfsstoffe.

5.  Trockenpräparat bestehend aus der Einschlussverbindung der in Wasser schwerlöslichen, antiepileptisch wirksamen Verbindung Carbamazepin oder Oxcarbazepin, dem verätherten, wasserlöslichen $\gamma$-Cyclodextrinderivat und gegebenenfalls für die Herstellung von Trockenpräparaten geeigneten wasserlöslichen

9

Hilfsstoffen.

6. Trockenpräparat gemäss Anspruch 5 bestehend aus der Einschlussverbindung von Carbamazepin oder Oxcarbazepin in Hydroxypropyl-γ-cyclodextrin, worin die Hydroxygruppe des Hydroxypropylrests sich in 2- oder 3-Stellung dieses Rests befindet.

7. Verfahren zur Herstellung einer pharmazeutischen Zusammensetzung gemäss Anspruch 1, dadurch gekennzeichnet, dass man
   α) als Löslichkeitsvermittler ein veräthertes, wasserlösliches γ-Cyclodextrinderivat in der Trägerflüssigkeit löst, Carbamazepin oder Oxcarbazepin oder einen Metaboliten davon und gegebenenfalls weitere wasserlösliche, pharmazeutische Hilfsstoffe, zur wässrigen Trägerflüssigkeit zusetzt und gegebenenfalls die Lösung homogenisiert ; oder
   β) ein Trockenpräparat enthaltend Carbamazepin, Oxcarbazepin oder einen Metaboliten davon und das verätherte, wasserlösliche γ-Cyclodextrinderivat herstellt und gegebenenfalls für die Herstellung von Trockenpräparaten geeignete wasserlösliche Hilfsstoffe hinzufügt und das Trockenpräparat in der Trägerflüssigkeit auflöst.

8. Verwendung eines verätherten, wasserlöslichen γ-Cyclodextrinderivats zur Herstellung einer wässrigen, intravenös applizierbaren Lösung von Carbamazepin oder Oxcarbazepin.

9. Stabile intravenöse Lösungen von Carbamazepin oder Oxcarbazepin zur Anwendung in einem Verfahren zur Behandlung des menschlichen oder tierischen Körpers.

## Patentansprüche für folgenden Vertragsstaaten : ES und GR

1. Verfhraren zur Herstellung einer pharmazeutischen Zusammensetzung für die intravenöse Applikation der in Wasser schwerlöslichen, antiepileptisch wirksamen Verbindungen Carbamazepin und Oxcarbazepin enthaltend :
   a) die in Wasser schwerlöslichen antiepileptisch wirksamen Verbindungen Carbamazepin und Oxcarbazepin sowie Metabolite davon ;
   b) als Löslichkeitsvermittler ein veräthertes wasserlösliches γ-Cyclodextrinderivat ;
   c) die für Injektionszwecke zubereitete Trägerflüssigkeit und gegebenenfalls weitere für die Herstellung von intravenösen Darreichungsformen geeignete Hilfsstoffe, dadurch gekennzeichnet, dass man
   α) als Löslichkeitsvermittler ein veräthertes, wasserlösliches γ-Cyclodextrinderivat in der Trägerflüssigkeit löst, Carbamazepin oder Oxcarbazepin oder einen Metaboliten davon und gegebenenfalls weitere wasserlösliche, pharmazeutische Hilfsstoffe, zur wässrigen Trägerflüssigkeit zusetzt und gegebenenfalls die Lösung homogenisiert ; oder
   β) ein Trockenpräparat enthaltend Carbamazepin, Oxcarbazepin oder einen Metaboliten davon und das verätherte, wasserlösliche γ-Cyclodextrinderivat herstellt und gegebenenfalls für die Herstellung von Trockenpräparaten geeignete wasserlösliche Hilfsstoffe hinzufügt und das Trockenpräparat in der Trägerflüssigkeit auflöst.

2. Verfhraen zur Herstellung einer pharmazeutischen Zusammensetzung gemäss Anspruch 1 enthaltend :
   a) Carbamazepin oder Oxcarbazepin ;
   b) als Löslichkeitsvermittler durch $C_1$-$C_6$-Alkyl, Carboxy-$C_1$-$C_6$-alkyl, $C_1$-$C_6$-Alkoxycarbonyl-$C_1$-$C_6$-alkyl und/oder Hydroxy-$C_2$-$C_6$-alkyl, wobei die Hydroxygruppe in einer höheren als der 1-Stellung des $C_2$-$C_4$-Alkylrests sich befindet, veräthertes γ-Cyclodextrin ;
   c) die Trägerflüssigkeit und gegebenenfalls weitere wasserlösliche, pharmazeutische Hilfsstoffe.

3. Verfahren zur Herstellung einer pharmazeutischen Zusammensetzung gemäss Anspruch 1 enthaltend :
   a) Carbamazepin oder Oxcarbazepin ;
   b) als Löslichkeitsvermittler durch Hydroxy-$C_2$-$C_4$-alkyl, wobei die Hydroxygruppe sich in einer höheren als der 1-Stellung des $C_2$-$C_4$-Alkylrests befindet, veräthertes γ-Cyclodextrin ;
   c) die Trägerflüssigkeit und gegebenenfalls weitere wasserlösliche, pharmazeutische Hilfsstoffe.

4. Verfahren zur Herstellung einer pharmazeutischen Zusammensetzung gemäss Anspruch 1 enthaltend :
   a) Carbamazepin oder Oxcarbazepin ;
   b) als Löslichkeitsvermittler Hydroxypropyl-γ-cyclodextrin, worin die Hydroxygruppe des Hydroxypropylrests sich in 2- oder in 3-Stellung dieses Rests befindet ;
c) die Trägerflüssigkeit und gegebenenfalls weitere wasserlösliche pharmazeutische Hilfsstoffe.

5. Verfahren zur Herstellung eines Trockenpräparats bestehend aus der Einschlussverbindung der in Wasser schwerlöslichen, antiepileptisch wirksamen Verbindung Carbamazepin oder Oxcarbazepin, dem verätherten, wasserlöslichen γ-Cyclodextrinderivat und gegebenenfalls für die Herstellung von Trockenpräparaten geeigneten wasserlöslichen Hilfsstoffen.

6. Verfahren zur Herstellung eines Trockenpräparats gemäss Anspruch 5 bestehend aus der Einschlussverbindung von Carbamazepin oder Oxcarbazepin in Hydroxypropyl-γ-cyclodextrin, worin die Hydroxygruppe des Hydroxypropylrests sich in 2- oder 3-Stellung dieses Rests befindet.

Europäisches
Patentamt

**EUROPÄISCHER RECHERCHENBERICHT**

Nummer der Anmeldung

EP    90 81 1002

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-A-3813015 (DESITIN ARZNEIMITTEL GMBH) <br> * das ganze Dokument * <br> --- | 1-9 | A61K31/55 <br> A61K47/48 <br> A61K9/18 |
| Y | EP-A-197571 (JANSSEN PHARMACEUTICA N.V.) <br> * das ganze Dokument * <br> --- | 1-9 | |
| A | EP-A-50589 (CIBA-GEIGY AG) <br> * das ganze Dokument * <br> --- | 1-9 | |
| A | US-A-4596795 (JOSEF PITHA) <br> * das ganze Dokument * <br> ----- | 1-9 | |
| | | | **RECHERCHIERTE SACHGEBIETE (Int. Cl.5)** <br><br> A61K |

Der vorliegende Recherchenbericht wurde für alle Patentansprüche erstellt

| Recherchenort | Abschlußdatum der Recherche | Prüfer |
|---|---|---|
| BERLIN | 18 MAERZ 1991 | SIATOU, E. |